# EUROPEAN PATENT SPECIFICATION

(11) **EP 2 963 026 B1**
(45) Date of publication and mention of the grant of the patent: **29.04.2020**
(21) Application number: 14756865.3
(22) Date of filing: 26.02.2014
(51) Int. Cl.: C07D 307/24, C07D 491/107, C07F 7/10

(54) **SUBSTITUTED (R)-3-(4-METHYLCARBAMOYL-3-FLUOROPHENYLAMINO)TETRAHYDROFURAN-3-ENECARBOXYLIC ACID (VARIANTS) AND ESTER THEREOF, METHOD FOR PRODUCING AND USING SAME**
SUBSTITUIERTE (R)-3-(4-METHYLCARBAMOYL-3-FLUORPHENYLAMINO-)TETRAHYDROFURAN-3-ENECARBONSÄURE (VARIANTEN) UND ESTER DAVON, VERFAHREN ZUR HERSTELLUNG UND VERWENDUNG DAVON
ACIDE SUBSTITUÉ (R)-3-(4-MÉTHYLCARBAMOYL-3-FLUOROPHÉNYLAMINO)TÉTRAHYDROFURAN-3-ÈNE-CARBONÉ (ET VARIANTES) ET SON ÉTHER, PROCÉDÉ DE PRODUCTION ET D'UTILISATION

(30) Priority: 27.02.2013 RU 2013108672
(43) Date of publication of application: 06.01.2016
(73) Proprietor: R-Pharm International, LLC, 123154 Moscow (RU)
(72) Inventor: IVACHTCHENKO, Alexandre Vasilievich, Encinitas, CA 92024 (US); MITKIN, Oleg Dmitrievich, Khimki Moskovskaya obl. 141400 (RU); KRAVCHENKO, Dmitry Vladimirovich, Khimki Moskovskaya obl. 141100 (RU); VOROBEV, Anton Aleksandrovich, Aleksandrov Vladimirskaya obl. 601655 (RU); TRIFILENKOV, Andrey Sergeevich, Moscow 125473 (RU)
(74) Representative: Zellentin & Partner mbB Patentanwälte
(86) International application number: PCT/RU2014/000122
(87) International publication number: WO 2014/133416

(56) References cited:
- EP-A1- 2 597 086
- WO-A1-2006/124118
- WO-A1-2010/118354
- WO-A1-2011/106570
- WO-A2-2007/127010
- RU-C1- 2 434 851

## Description

### Field of invention

The present invention relates to novel chemical compounds - intermediate products in synthesis of androgen receptor inhibitors which are of interest as anticancer medicaments. The subject of the invention is also methods for preparation of novel compounds - androgen receptor inhibitors.

### Background of the invention

There are known inhibitors of androgen receptors, among them Enzalutamide, previously known as MDV3100, which at the end of 2012 entered the market as Xtandi and which is intended for prostate cancer treatment [WO 2011/106570 A1] and ARN-509, successfully passing phase II of clinical trial as a drug for prostate cancer treatment [WO 2008/119015 A2].

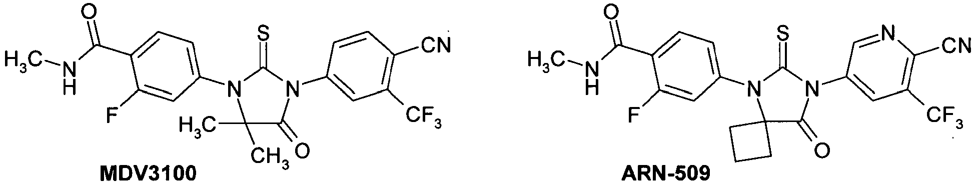

### Disclosure of the invention

In context of the invention, terms are generally defined as follows:
**"Alkyl"** means an aliphatic hydrocarbon straight or branched group with 1-5 carbon atoms in chain, preferably 1-4 carbon atoms (C₁-C₄ alkyl). Branched alkyl means that the alkyl chain has one or more substituents, preferably C₁-C₄ alkyl, C₁-C₄ alkyloxymethyl, trimethylsilylethoxymethyl.
**"Amino group"** means Rₖ^{a}Rₖ₊₁^{a}N- group substituted or not with "amino group substituent" Rₖ^{a} and Rₖ₊₁^{a}, the meanings of which is defined in this section, for example, amino (H₂N-), methylamino, dimethylamino, N-(trimethylsilylethoxymethyl)methylamino, diethylamino, pyrrolidine, morpholine, benzylamino, or phenethylamino.
**"Aminocarbonyl"** means C(=O)NRₖ^{a}Rₖ₊₁^{a} -group, substituted or not with optionally the same "carbamoyl substituents" Rₖ^{a} and Rₖ₊₁^{a}, including hydrogen, alkenyl, alkyl, aryl, heteroaryl, heterocyclyl, the meanings of which are defined in this section.
**"Aryl"** means an aromatic monocyclic or polycyclic system, including 6-14 carbon atoms, preferably 6-10 carbon atoms. Aryl may contain one or more "cyclic system substituents" of the same or different structure. Representatives of aryl groups are phenyl or naphthyl, substituted phenyl or substituted naphthyl. Aryl may be annelated with non-aromatic cyclic system or heterocycle.
**"Acylamino"** means acyl-NH-group, for which the meaning of acyl is defined in this section.
**"Halogen"** means fluorine, chlorine, bromine and iodine. Fluorine, chlorine, and bromine are preferable.
**"Heterocyclyl"** means aromatic or non-aromatic saturated monocyclic or polycyclic system, including 3-10 carbon atoms, preferably 5-6 carbon atoms, wherein one or more carbon atoms are substituted with heteroatom such as nitrogen, oxygen, or sulfur. Prefix "aza", "oxa", or "thia" before heterocyclyl means the presence of atoms N, O, or S in the cyclic system, respectively. Heterocyclyl may have one or more "cyclic system substituents" of the same or different structure. N- and S- atoms, which are in heterocyclyl may be oxidized to N-oxide, S-oxide, and S-dioxide. Representatives of heterocyclyls are tetrahydrofuran, piperidine, pyrrolidine, piperazine, morpholine, thiomorpholine, thiazolidine, 1,4-dioxane, tetrahydrothiophene and others.
**"Substituent"** means a chemical radical, which is attached to scaffold (fragment), for example, "alkyl substituent", "amino group substituent", "hydroxy group substituent", "carbamoyl substituent", "cyclic system substituent", the meanings of which are defined in this section.
**"Amino group substituent"** means a substituent attached to amino group. Amino group substituent represents hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl. Preferably, substituents are selected from hydrogen, trimethylsilylethoxymethyl, methyl, substituted fluorophenyl, substituted alkyl.
**"Carboxyl substituent"** means a substituent attached to O-atom of carboxyl group, the meaning of which is defined in this section. Carboxyl substituent is alkyl, cycloalkyl, aryl, heteroaryl, heterocyclyl, alkoxycarbonylalkyl, aralkoxycarbonylalkyl, heteroaralkyloxycarbonylalkyl, or Rₖ^{a}Rₖ₊₁^{a}N- alkyl, Rₖ^{a}Rₖ₊₁^{a}NC(=O)-alkyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl. Preferably, "carboxyl substituents" are alkyl, cycloalkyl, aryl, heteroaryl, heterocyclyl, alkoxycarbonylalkyl, aralkoxycarbonylalkyl, heteroaralkyloxycarbonylalkyl, or Rₖ^{a}Rₖ₊₁^{a}N- alkyl, Rₖ^{a}Rₖ₊₁^{a}NC(=O)-alkyl, arylheterocyclenyl, annelated arylheterocyclyl. Preferable substituents are C₁-C₄alkyl (methyl, ethyl, propyl, *iso*propyl, butyl, *tert*-butyl and others).
**"Carboxyl"** means HOC(=O) - (carboxyl) group.
   Recently have been prepared effective androgen receptor inhibitors representing 4-[3-(3-trifluoromethyl-4-cyanophenyl)-4-oxo-2-thioxo-7-oxa-1,3-diazaspiro[4.4]non-1-yl]-2-fluoro-N-methylbenzamide **A1** and (R)- **A2** and (S)- **A3** stereoisomers thereof [RU patent 2434851, publ. 27.11.2011; WO 2012/011840, publ. 26.01.2012].

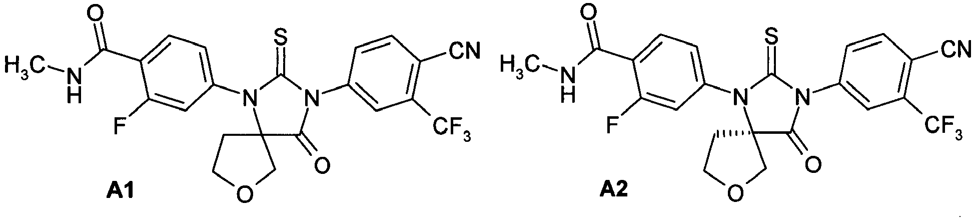

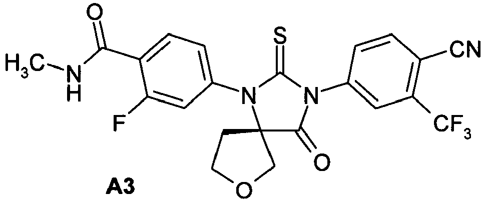

Synthesis of 4-[3-(3-trifluoromethyl-4-cyanophenyl)-4-oxo-2-thioxo-7-oxa-1 ,3-diazaspiro[4.4]non-1-yl]-2-fluoro-N-methylbenzamide **A1** was carried out by interaction of 4-thioisocyanate-2-(trifluoromethyl)benzonitrile **B1** with 4-(3-cyano-tetrahydrofuran-3-ylamino)-2-fluoro-N-methylbenzamide **B2** in dimethylformamide in microwave oven at 100°C for 12 hours according to Scheme 1. Product **A1** of the reaction was isolated from the reaction mixture by means of high pressure liquid chromatography and divided into stereoisomers **A2** and **A3** using high pressure liquid chromatography on Chiralpak HD-H 25x1cm (Chiral Technologies Inc., USA).

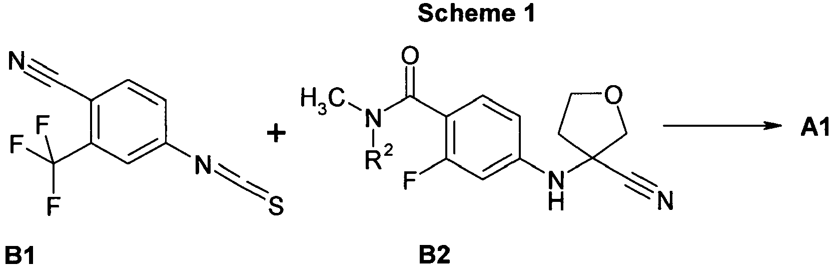

Separation of the final product **A1** and optical isomers **A2** and **A3** thereof by means of high pressure liquid chromatography, including usage of chiral chromatographic columns, makes the process of their preparation more difficult and expensive. Therefore, the search of novel intermediates for synthesis of androgen receptor inhibitors **A1, A2,** and **A3** is an actual task.

The subject of the present invention is unknown before compounds 3-(4-methylcarbamoyl-3-fluorophenylamino)tetrahydrofuran-3-encarboxylic acid esters of the general formula **1** and stereoisomers thereof, 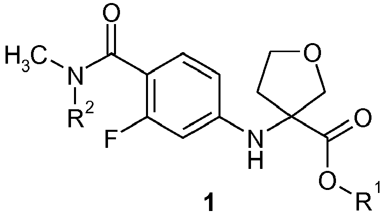 wherein:
R¹ = C₁-C₄alkyl;
R² = H, CH₂OCH₂CH₂Si(CH₃)₃.

The more preferable compounds of the general formula **1** are methyl (R)-3-(4-methylcarbamoyl-3-fluorophenylamino)tetrahydrofuran-3-encarboxylate **1(2)** or butyl (R)-3-(4-methylcarbamoyl-3-fluorophenylamino)tetrahydrofuran-3-encarboxylate **1(3)** 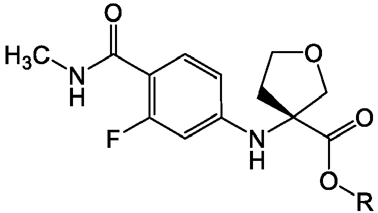 **1(2):** R = CH₃, **1(3):** R = C₄H₉

The subject of the present invention is a method for preparation of compounds of the general formula **1** and stereoisomers **1(2)-1(3)** thereof by interaction of 4-bromo-2-fluoro-N-methylbenzamide of the general formula **2** with 3-aminotetrahydrofuran-3-carboxylic acid ester of the general formula **3.2** or stereoisomers thereof 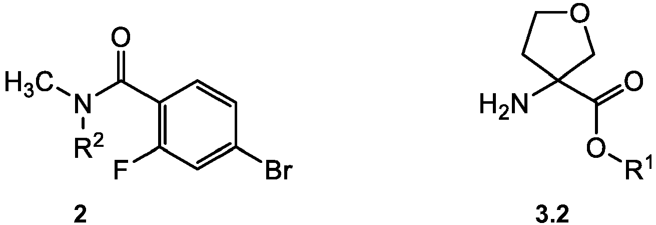 **2:** R² = H, CH₂OCH₂CH₂Si(CH₃)₃. **3.2:** R¹ = C₁-C₄alkyl.

The best results were achieved when compounds **2** reacted with compound **3.2** in the medium of water dimethylformamide in the presence of copper iodide (I), potassium carbonate, triethylamine and 2-acetylcyclohexanone.

The subject of the present invention are also intermediates in the synthesis of 4-[3-(3-trifluoromethyl-4-cyanophenyl)-4-oxo-2-thioxo-7-oxa-1,3-diazaspiro[4.4]non-1-yl]-2-fluoro-N-methylbenzamides **A1, A2,** and **A3,** representing compounds of the general formula **1** or stereisomers thereof 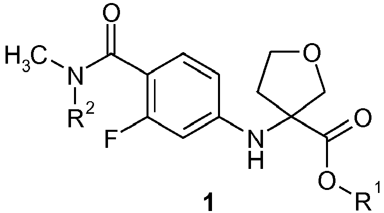 R² = H, CH₂OCH₂CH₂Si(CH₃)₃; R¹ = C₁-C₄alkyl.

The more preferable intermediates are methyl (R)-3-(4-methylcarbamoyl-3-fluorophenylamino)tetrahydrofuran-3-encarboxylate **1(2)** and butyl (R)-3-(4-methylcarbamoyl-3-fluorophenylamino)tetrahydrofuran-3-encarboxylate **1(3).** 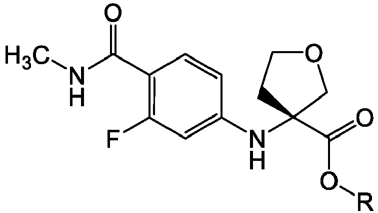 **1(2):** R = CH₃, **1(3):** R = C₄H₉

The subject of the present invention is also a method for preparation of 4-[3-(3-trifluoromethyl-4-cyanophenyl)-4-oxo-2-thioxo-7-oxa-1,3-diazaspiro[4.4]non-1-yl]-2-fluoro-N-methylbenzamides **A1, A2,** and **A3,** consisting in interaction of a compound of the general formula **1** or stereoisomer thereof with 4-isocyanato-2-trifluoromethylbenzonirile **B1** in a mixture of dimethyl sulphoxide and ethyl acetate at elevated temperature according to Scheme 2. 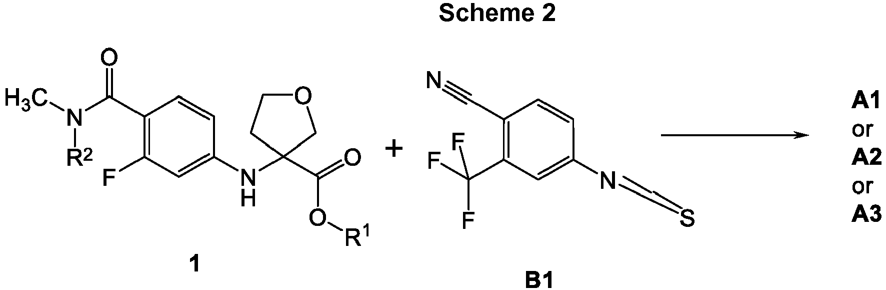
R² = H, CH₂OCH₂CH₂Si(CH₃)₃;
R¹ = C₁-C₄ alkyl.

The best results were achieved when the reaction was carried out in argon atmosphere in the mixture of dimethyl sulphoxide and ethyl acetate taken in ratio 1:2, respectively.

Utilization of this invention allows to simplify the process of preparation of androgen receptor inhibitors **A1, A2,** and **A3,** increase the yield of the products to 70% and eliminate the use of chiral chromatography.

### The best embodiment of the invention

Below the invention is described by means of specific examples which illustrate, but not limit the scope of the invention.

### Example 1. The general method for preparation of 3-(4-methylcarbamoyl-3-fluorophenylamino)tetrahydrofuran-3-encarboxylic acid, its ester of the general formula 1 and stereoisomer thereof.

In a round bottom flask provided with magnetic stirrer, reflux condenser and oil bath with a temperature controller, was placed 1.2 eq. of acid **3.1** or its ester of the general formula **3.2,** 1 eq. of 4-bromo-2-fluoro-N-methylbenzamide of the general formula **2,** 0.35 g (0.15 eq.) of copper iodide (I), 6.76 g (4 eq.) of potassium carbonate, 6 ml of water, 27.5 ml of dimethylformamide, and 0.2 ml of triethylamine. After 10 min of stirring 0.35 g (0.2 eq.) of 2-acetylcyclohexanone was added. The reaction mixture was stirred at 100°C for two days. After the reaction was completed solvents were distilled *in vacuo.* It gave ester of the general formula **1,** wherein R¹ = C₁-C₄alkyl, which was mixed with water and acidified with hydrochloric acid (conc.) till pH about 2-3. In 20-30 min. at stirring a solid was formed, which was filtered off, washed with water, and dried. Then it was washed with ether. It gave an acid of the general formula **1,** where R¹ = H. Yield is above 80%.

### (R)-3-{4-[Methyl-(2-trimethylsilylethoxymethyl)carbamoyl]-3-fluorophenylamino}tetra hydrofuran-3-carboxylic acid 1 [R¹ = H, R² = CH₂OCH₂ CH₂Si(CH₃)₃] (reference example).

LC MS m/e 413 (M+1); ¹H NMR (DMSO, 400 MHz) δ 12.85 (br. s, 1H), 7.05 (t, 1H), 6.97 (br. s, 1H), 6.30 (m, 1H), 6.15 (d, 1H), 4.84 (s, 1H), 4.60 (s, 1H), 4.09 (d, 1H), 3.88 (t, 3H), 3.50 (br. s, 1H), 3.32 (br. s, 1H), 2.90 (m, 3H), 2.56 (m, 1H), 2.16 (m, 1H), 0.87 (br. s, 1H), 0.76 (br. s, 1H), 0.00 (m, 9H).

***(R)-3-(4-Methylcarbamoyl-3-fluorophenylamino)tetrahydrofuran-3-encarboxylic acid 1(1) (reference example),*** LC MS m/e 283 (M+1); ¹H NMR (DMSO, 400 MHz) δ 13.01 (br. s, 1H), 7.68 (s, 1H), 7.46 (t, 1H), 7.13 (s, 1H), 6.31 (d, 1H), 6.11 (d,1H), 4.09 (d, 1H), 3.86 (m, 3H), 2.71 (m, 4H), 2.14 (m, 1H).

### Example 2. Method for preparation of methyl (R)-3-(4-methylcarbamoyl-3-fluorophenylamino)tetrahydrofuran-3-encarboxylate 1(2).

**Process A.** 500 mg of (R)-3-(4-Methylcarbamoyl-3-fluorophenylamino)tetrahydrofuran-3-encarboxylic acid **1(1)** and 294 mg of potassium carbonate (1.2 eq.) were mixed together in 4 ml of DMF. The mixture was heated to 30°C and 1.2 eq. of methyl iodide was introduced. The temperature was raised to 40°C and mixture was kept for one hour. Then 40 ml of water was added, and mixture was heated to 60°C. The mixture was filtered, water layer was extracted with chloroform (2 x 50ml). Combined organic layers were washed with saturated water solution of sodium chloride, the solvent was evaporated *in vacuo.* The product was mashed with ether. It gave 372 mg (70%) of product **1(2).**
**Process B.** To a solution of 10 g of (R)-3-(4-Methylcarbamoyl-3-fluorophenylamino)tetrahydrofuran-3-encarboxylic acid **1(1)** in acetonitrile was added 6.89 g (1.2 eq.) of carbodiimide. The mixture was stirred at 60°C for 1 h, and 20 ml of methanol was introduced. The mixture was left stirring for 2-3 hours. Then solvents were evaporated *in vacuo,* water was added to the residue. Precipitated solid was filtered off and dried. Yield of ester **1(2)** was 7.5 g (71%).
**Process C.** 15.5 g of (R)-3-(4-Methylcarbamoyl-3-fluorophenylamino)tetrahydrofuran-3-encarboxylic acid **1(1)** was dissolved in 150 ml of methanol. The flask was cooled with ice and water and the excess of thionyl chloride (1.2 eq.) was added dropwise to the solution for 10 min. Then the reaction mixture was brought to boiling and refluxed for 4 hours. After that the solvent and the excess of thionyl chloride were evaporated *in vacuo.* The residue was dissolved in 200 ml of chloroform, washed with saturated solution of sodium hydrocarbonate (50 ml), and saturated solution of sodium chloride (50 ml). Chloroform was evaporated *in vacuo,* ether was added to the residue, and the product was filtered off. Yield of ester **1(2)** was 12.5 g (77%).

***Methyl (R)-3-(4-methylcarbamoyl-3-fluorophenylamino)tetrahydrofuran-3-encarboxylate 1(2),*** LC MS m/e 297 (M+1); ¹H NMR (CDCl₃, 400 MHz) δ 7.94 (t, 1H), 6.59 (br. s, 1H), 6.38 (d, 1H0, 6.20 (d, 1H), 4.80 (br. s, 1H), 4.19 (d, 1H), 4.05 (m,3H), 3.76 (s, 3H), 3.01 (d, 3H), 2.51 (m,1H), 2.19 (m,1H).

### Example 3. Method for preparation of butyl (R)-3-(4-methylcarbamoyl-3-fluorophenylamino)tetrahydrofuran-3-encarboxylate 1(3).

0.55g of Butyl (R)-3-aminotetrahydrofuran-3-carboxylate **3.2(1),** 0.68 g (1 eq.) of 4-bromo-2-fluoro-N-methyl-N-{[2-(trimethylsilyl)ethoxy]methyl}benzamide **2.1,** 0.27 g (0.1eq.) of Pd*DBA, 0.37 g (0.2 eq.) of BINAP were mixed together in 10 ml of dioxane. The mixture was refluxed in argon atmosphere for 2 days. After that it was poured into water, extracted with chloroform, extract was washed with saturated solution of sodium chloride. Chloroform was evaporated *in vacuo,* the residue was subjected to chromatography on silica. It gave butyl (R)-3-(4-methylcarbamoyl-3-fluorophenylamino)tetrahydrofuran-3-encarboxylate **1(3),** yield ∼ 10%, LC MS m/e 339 (M+1).

**Example 4. Methods for preparation of 4-[3-(3-trifluoromethyl-4-cyanophenyl)-4-oxo-2-thioxo-7-oxa-1,3-diazaspiro[4.4]non-1-yl]-2-fluoro-N-methylbenzamide A1, 4-[(R)-3-(3-trifluoromethyl-4-cyanophenyl)-4-oxo-2-thioxo-7-oxa-1,3-diazaspiro[4.4]non-1-yl]-2-fluoro-N-methylbenzamide A2, 4-[(S)-3-(3-trifluoromethyl-4-cyanophenyl)-4-oxo-2-thioxo-7-oxa-1,3-diazaspiro[4.4]non-1-yl]-2-fluoro-N-methylbenzamide A3, 4-[(R)-3-(3-trifluoromethyl-4-cyanophenyl)-4-oxo-2-thioxo-7-oxa-1,3-diazaspiro[4.4]non-1-yl]-2-fluoro-N-methyl-N-(2-trimethylsilylethoxymethyl)benzamide A4. Process A *(reference example).*** In a round bottom flask of 250 ml, provided with magnetic stirrer, reflux condenser, calcium chloride tube, and an oil bath with temperature controller IKA^{R}- ETS-D4, 10.7 g of (R)-3-{4-methyl-(2-trimethylsilylethoxymethyl)carbamoyl]-3-fluorophenylamino}tetrahydrofuran-3-carboxylic acid **1** [R¹=H, R²= CH₂OCH₂CH₂Si(CH₃)3] and 1.5 eq. of 4-thioisocyanate-2-(trifluoromethyl)benzonitrile **B1** in 100 ml of pyridine were placed. The reaction mixture was stirred for 48 hours at 80°C. When the reaction was completed (LCMS control) the most part of pyridine was evaporated *in vacuo.* The residue was dissolved in ethyl acetate and filtered through a layer of silica, the solvent was distilled *in vacuo,* ethanol was added to the residue. Precipitated solid was filtered off and dried. It gave 4.84 g of 4-[(R)-3-(3-trifluoromethyl-4-cyanophenyl)-4-oxo-2-thioxo-7-oxa-1,3-diazaspiro[4.4]non-1-yl]-2-fluoro-N-methyl-N-(2-trimethylsilylethoxymethyl)benzamide **A4,** LC MS m/e 623 (M+1); ¹H NMR (CDCl₃, 400 MHz) δ 8.01 (d, 1H), 7.98 (s, 1H), 7.85 (d, 1H), 7.57 (m, 1H), 7.22 (m, 2H), 4.97 (s, 1H0, 4.55 (s, 1H), 4.32 (d, 1H), 4.06 (d, 1H), 3.86 (qv, 1H), 3.66 (m, 1H), 3.57 (t, 1H), 3.26 (t, 1H), 3.10 (s, 2H), 2.89 (s, 1H), 2.64 (m, 1H), 2.41 (m, 2H), 0.91 (t, 1H), 0.77 (t, 1H), 0.06 (s, 3H), 0.00 (s, 6H).

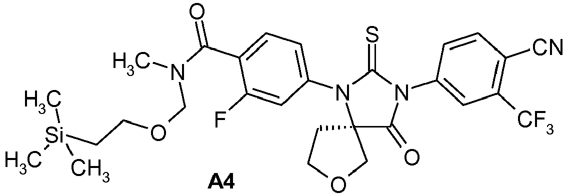

4.8 g of 4-[(R)-3-(3-Trifluoromethyl-4-cyanophenyl)-4-oxo-2-thioxo-7-oxa-1,3-diazaspiro[4.4]non-1-yl]-2-fluoro-N-methyl-N-(2-trimethylsilylethoxymethyl)benzamide **A4** were stirred with 15 ml of trifluoroacetic acid in 30 ml of methylene chloride at room temperature for 2-3 hours. After the reaction was completed (no starting compound **A4** according to LCMS control), the reaction mixture was evaporated *in vacuo,* the residue was purified by chromatography on silica. A system of chloroform-methanol (60:1) was used as eluent. The fraction with R_{f} = 0.4 was collected. It gave 4-[(R)-3-(3-trifluoromethyl-4-cyanophenyl)-4-oxo-2-thioxo-7-oxa-1,3-diazaspiro[4.4]non-1-yl]-2-fluoro-N-methylbenzamide **A2,** 78%, LC MS m/e 493 (M+1); ¹H NMR (CDCl₃, 400 MHz) δ 8.30 (t, 1H), 8.06 (d, 1H), 7.96 (s, 1H), 7.83 (d, 1H), 7.32 (d, 1H), 7.23 (d, 1H), 6.71 (m, 1H), 4.93 (d, 1H), 4.16 (d, 1H), 3.95 (qv, 1H), 3.42 (qv, 1H), 3.08 (d, 3H), 2.73 (m, 1H), 2.47 (m, 1H).

**Process B *(reference example).*** 5.09 g of (R)-3-(4-Methylcarbamoyl-3-fluorophenylamino)tetrahydrofuran-3-encarboxylic acid **1(1)** and 1.5 eq. of 4-thioisocyanate-2-(trifluoromethyl)benzonitrile **B1** in 50 ml of pyridine were stirred at 80°C for 48 hours. After the reaction was completed (LCMS control), the most part of pyridine was evaporated *in vacuo.* The residue was subjected to chromatography on silica. A system of chloroform-methanol (60:1) was used as eluent. The fraction with R_{f} = 0.6 was collected. It gave 630 mg (yield 7.6%) of product **A2** with purity 99.2%, LC MS m/e 493 (M+1); ¹H NMR (CDCl₃, 400 MHz) δ 8.30 (t, 1H), 8.06 (d, 1H), 7.96 (s, 1H), 7.83 (d, 1H), 7.32 (d, 1H), 7.23 (d, 1H), 6.71 (m, 1H), 4.93 (d, 1H), 4.16 (d, 1H), 3.95 (qv, 1H), 3.42 (qv, 1H), 3.08 (d, 3H), 2.73 (m, 1H), 2.47 (m, 1H).

*Note.* Carrying out the reaction: in a microwave reactor in pyridine (70°C, 3 h) gave final product **A2** with yield 10%; in acetonitrile in the presence of K₂CO₃ (60°C, 12 h) gave final product **A2** with yield 8%; in acetonitrile in the presence of (C₂H₅)₃N in argon atmosphere (75°C, 3 h) gave final product **A2** with yield 28%.

**Process C.** 1.05 g of methyl (R)-3-(4-methylcarbamoyl-3-fluorophenylamino) tetrahydrofuran-3-encarboxylate **1(2)** and 1.62 g (2eq.) of 4-thioisocyanate-2-(trifluoromethyl)benzonitrile **B1** in a mixture of 252 µl (1 eq.) of DMSO and 692 µl (2 eq.) of ethyl acetate were stirred together at 85°C for 48 hours. After the reaction was completed (LCMS and TLC control) the solvents were evaporated *in vacuo.* The residue was subjected to flash-chromatography on silica. Chloroform was used as eluent. Fraction with R_{f} = 0.6 was collected (TLC was in system chloroform-methanol 60:1), the solvent was evaporated *in vacuo,* the residue was crystallized from ethanol. It gave product **A2** with yield 60-70% and 99.5% of basic material, LC MS m/e 493 (M+1); ¹H NMR (CDCl₃, 400 MHz) δ 8.30 (t, 1H), 8.06 (d, 1H), 7.96 (s, 1H), 7.83 (d, 1H), 7.32 (d, 1H), 7.23 (d, 1H), 6.71 (m, 1H), 4.93 (d, 1H), 4.16 (d, 1H), 3.95 (qv, 1H), 3.42 (qv, 1H), 3.08 (d, 3H), 2.73 (m, 1H), 2.47 (m, 1H).

In analogous manner 4-[(S)-3-(3-trifluoromethyl-4-cyanophenyl)-4-oxo-2-thioxo-7-oxa-1,3-diazaspiro[4.4]non-1-yl]-2-fluoro-N-methylbenzamide **A3** was prepared, starting from the corresponding (S)-3-(4-methylcarbamoyl-3-fluorophenylamino)tetrahydrofuran-3-encarboxylic acid of formula 1, yield 60-65%, LC MS m/e 493 (M+1).

In analogous manner 4-[3-(3-trifluoromethyl-4-cyanophenyl)-4-oxo-2-thioxo-7-oxa-1,3-diazaspiro[4.4]non-1-yl]-2-fluoro-N-methylbenzamide **A1** was prepared, starting from the corresponding 3-(4-methylcarbamoyl-3-fluorophenylamino)tetrahydrofuran-3-encarboxylic acid of formula **1,** yield 60-65%, LC MS m/e 493 (M+1).

### Industrial applicability

The invention could be used in medicine, veterinary, biochemistry.

## Claims

1. Compounds of the general formula **1** or stereoisomers thereof, 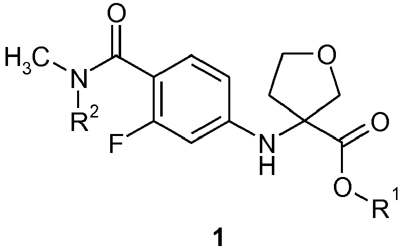 wherein:
R¹ = C₁-C₄alkyl;
R² = H, CH₂OCH₂CH₂Si(CH₃)₃.

2. Compounds according to claim **1,** representing methyl (R)-3-(4-methylcarbamoyl-3-fluorophenylamino)tetrahydrofuran-3-encarboxylate **1(2)** or butyl (R)-3-(4-methylcarbamoyl-3-fluorophenylamino)tetrahydrofuran-3-encarboxylate **1(3)** 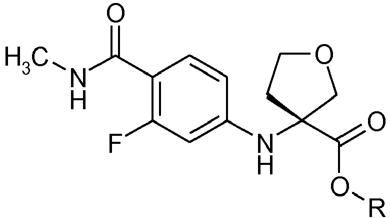 1(2): R = CH₃, 1(3): R = C₄H₉

3. A method for preparation of compounds of the general formula **1** or stereoisomers thereof 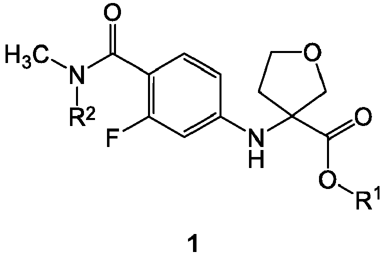 wherein:
R¹ = C₁-C₄alkyl;
R² = H, CH₂OCH₂CH₂Si(CH₃)₃;
by simultaneous interaction of 4-bromo-2-fluoro-N-methylbenzamide of the general formula **2,** K₂CO₃, 3-aminotetrahydrofuran-3-carboxylic acid ester of the general formula **3.2** or stereoisomers thereof 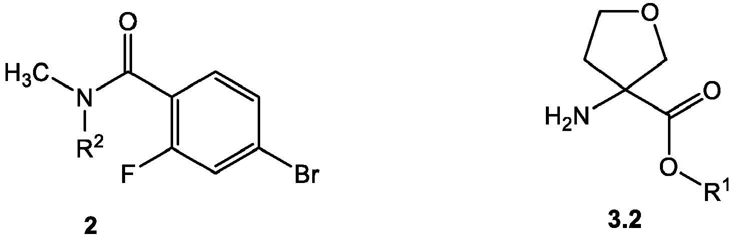
**2:** R² = H, CH₂OCH₂CH₂Si(CH₃)₃; **3.2:** R¹ = C₁-C₄alkyl;
in DMF, **characterized in that** copper iodide (I), water and triethylamine were added to the reaction mixture concurrently.

4. Method for preparation of 4-[3-(3-trifluoromethyl-4-cyanophenyl)-4-oxo-2-thioxo-7-oxa-1,3-diazaspiro[4.4]non-1-yl]-2-fluoro-N-methylbenzamides **A1, A2,** and **A3** 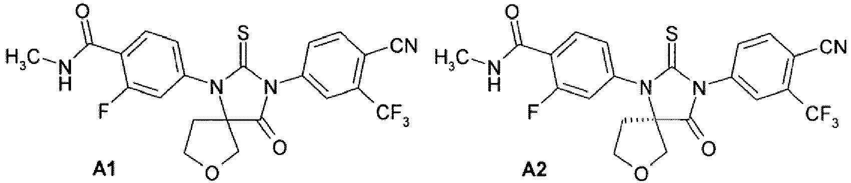 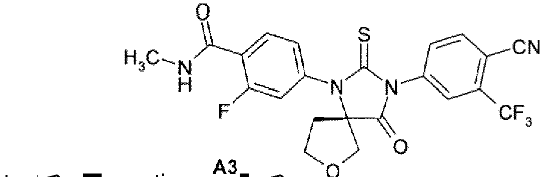 consisting in reacting a compound of the general formula **1** or stereoisomer thereof, 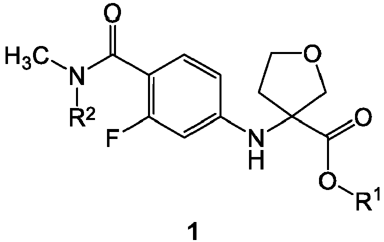 wherein:
R¹ = C₁-C₄ alkyl;
R² = H, CH₂OCH₂CH₂Si(CH₃)₃;
prepared according to claim 3,
with 4-isothiocyanate-2-trifluoromethylbenzonitrile **B1** 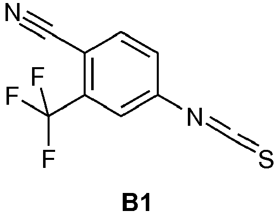 wherein the reagents are stirred in dimethyl sulphoxide and ethyl acetate mixture in ratio 1:2 at elevated temperature.

## Patentansprüche

1. Verbindungen der allgemeinen Formel **1** oder Stereoisomere davon 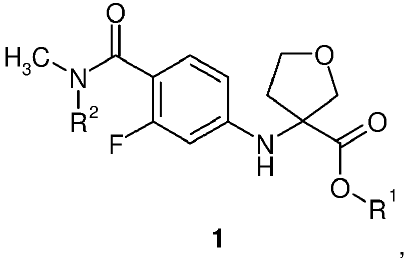 worin:
R¹ = C₁-C₄-Alkyl;
R² = H, CH₂OCH₂CH₂Si(CH₃)₃.

2. Verbindungen gemäß Anspruch **1,** die Methyl-(R)-3-(4-methylcarbamoyl-3-fluorphenylamino)tetrahydrofuran-3-encarboxylat **1(2)** oder Butyl-(R)-3-(4-methylcarbamoyl-3-fluorphenylamino)tetrahydrofuran-3-encarboxylat **1(3)** darstellen 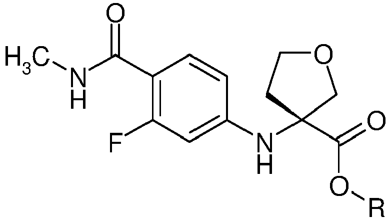 1(2): R = CH₃, 1(3): R = C₄H₉.

3. Verfahren zur Herstellung von Verbindungen der allgemeinen Formel **1** oder Stereoisomeren davon 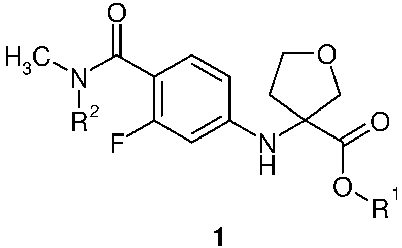 worin:
R¹ = C₁-C₄-Alkyl;
R² = H, CH₂OCH₂CH₂Si(CH₃)₃;
durch gleichzeitige Wechselwirkung von 4-Brom-2-Fluor-N-Methylbenzamid der allgemeinen Formel **2,** K₂CO₃, 3-Aminotetrahydrofuran-3-carbonsäure **3.1** oder deren Ester der allgemeinen Formel **3.2** oder Stereoisomere davon 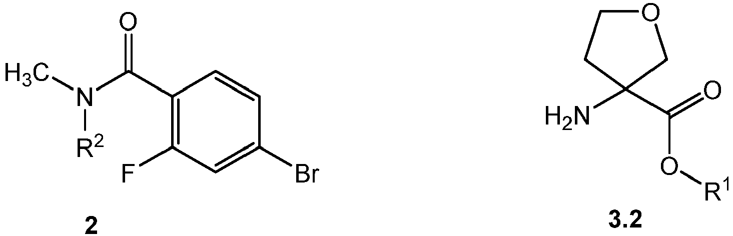
**2:** R² = H, CH₂OCH₂CH₂Si(CH₃)₃; **3.2:** R¹ = C₁-C₄-Alkyl;
in DMF, **dadurch gekennzeichnet, dass** dem Reaktionsgemisch gleichzeitig Kupfer(I)-iodid, Wasser und Triethylamin zugesetzt wurden.

4. Verfahren zur Herstellung von 4-[3-(3-Trifluormethyl-4-cyanophenyl)-4-oxo-2-thioxo-7-oxa-1,3-diazaspiro[4.4]non-1-yl]-2-fluor-N-methylbenzamiden **A1, A2** und **A3** 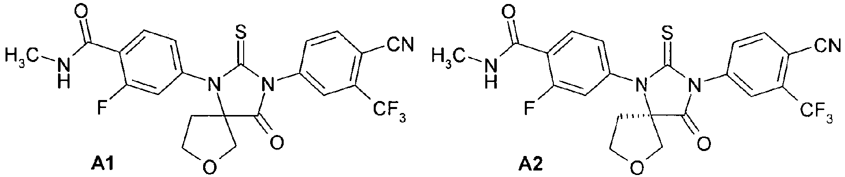 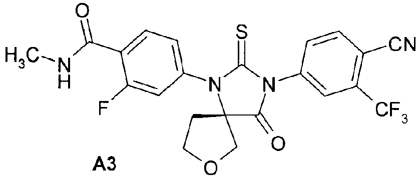 bestehend aus der Umsetzung einer Verbindung der allgemeinen Formel **1** oder eines Stereoisomers davon 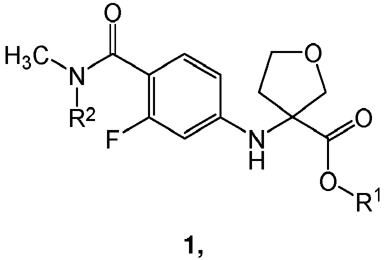 worin:
R¹ = C₁-C₄ alkyl;
R² = H, CH₂OCH₂CH₂Si(CH₃)₃;
hergestellt gemäß Anspruch 3,
mit 4-lsothiocyanat-2-trifluormethylbenzonitril **B1** 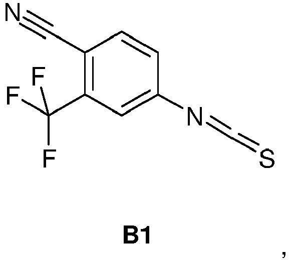 wobei die Reagenzien in einer Mischung aus Dimethylsulfoxid und Ethylacetat im Verhältnis 1:2 bei erhöhter Temperatur gerührt werden.

## Revendications

1. Composés répondant à la formule générale **1** ou leurs stéréo-isomères : 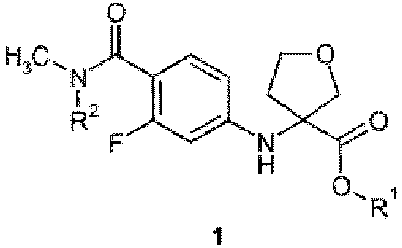 formule dans laquelle:
R¹ représente un groupe alkyle en C₁-C₄;
R² représente un atome d'hydrogène, un groupe CH₂OCH₂CH₂Si(CH₃)₃.

2. Composés selon la revendication 1, représentant le (R)-3-(4-méthylcarbamoyl-3-fluorophénylamino)tétrahydrofuran-3-ènecarboxylate de méthyle **1(2)** ou le (R)-3-(4-méthylcarbamoyl-3-fluorophénylamino)-tétrahydrofuran-3-ènecarboxylate de butyle **1(3)** 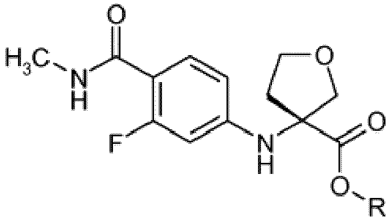 1(2): R représente un groupe CH₃, 1(3) : R représente un groupe C₄H₉.

3. Procédé pour la préparation de composés répondant à la formule générale **1** ou de leurs stéréo-isomères 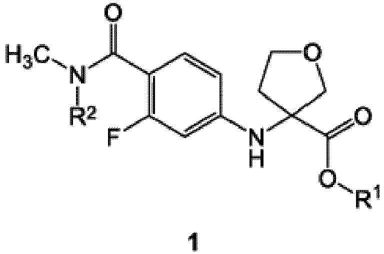 formule dans laquelle:
R¹ représente un groupe alkyle en C₁-C₄;
R² représente un atome d'hydrogène, un groupe CH₂OCH₂CH₂Si(CH₃)₃;
par l'intermédiaire d'une interaction simultanée du 4-bromo-2-fluoro-N-méthylbenzamide répondant à la formule générale **2,** du K₂CO₃, de l'ester de l'acide 3-aminotétrahydrofuran-3-carboxylique répondant à la formule générale **3.2** ou de ses stéréo-isomères 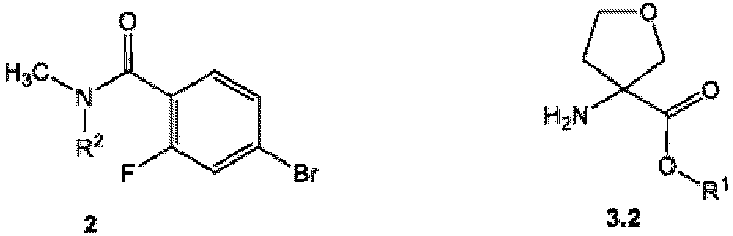
**2:** R² représente un atome d'hydrogène, un groupe CH₂OCH₂CH₂Si(CH₃)₃;
**3.2:** R¹ représente un groupe alkyle en C₁-C₄;
dans du DMF, **caractérisé en ce que** l'on ajoute de manière concourante au mélange réactionnel de l'iodure de cuivre(I), de l'eau et de la triéthylamine.

4. Procédé pour la préparation des 4-[3-(3-trifluorométhyl-4-cyanophényl)-4-oxo-2-thioxo-7-oxa-1,3-diazaspiro[4.4]non-1-yl]-2-fluoro-N-méthylbenzamides **A1, A2** et **A3:** 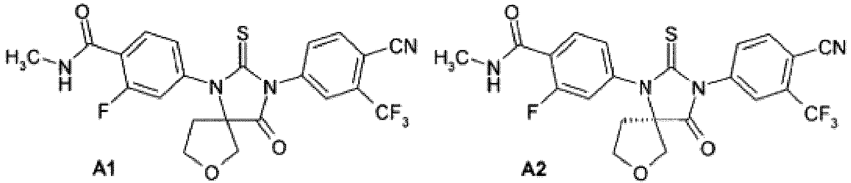 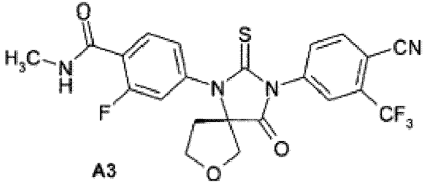 consistant à faire réagir un composé répondant à la formule générale 1 ou un de ses stéréo-isomères: 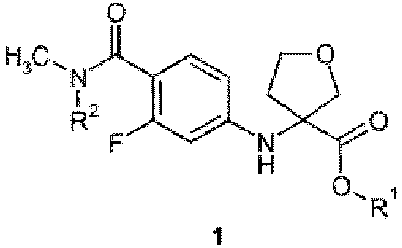 formule dans laquelle:
R¹ représente un groupe alkyle en C₁-C₄;
R² représente un atome d'hydrogène, un groupe CH₂OCH₂CH₂Si(CH₃)₃;
préparé conformément à la revendication 3;
avec du 4-isothiocyanate-2-trifluorométhylbenzonitrile **B1 :** 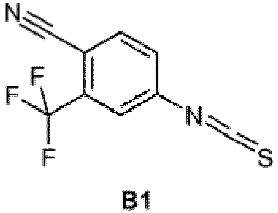 dans lequel on agite les réactifs dans un mélange de diméthylsulfoxyde et d'acétate d'éthyle dans un rapport 1:2 à une température élevée.
